# EUROPEAN PATENT APPLICATION

(11) **EP 4 763 068 A2**
(43) Date of publication of application: **24.06.2026**
(21) Application number: 25221445.7
(22) Date of filing: 08.12.2025
(51) Int. Cl.: A61B 5/024, A61B 5/352, A61B 5/00

(54) **DATA PREPROCESSING FOR PSYCHOPHYSIOLOGICAL STATE DETECTION**

(30) Priority: 20.12.2024 US 202418990455
(71) Applicant: Harman Becker Automotive Systems, Inc., Novi, MI 48377 (US)
(72) Inventor: SHISHALOV, Ivan Sergeevich, Novi, MI, 48377 (US); BAKHCHINA, Anastasiya Vladimirovna, Novi, MI, 48377 (US); ARUTYUNOVA, Karina, Novi, MI, 48377 (US)
(74) Representative: Westphal, Mussgnug & Partner, Patentanwälte mbB

(57) **Abstract**

Methods and systems are described for preprocessing and denoising R-R interval (RRI) data extracted from cardiac physiological data acquired via an echocardiograph (ECG) device. The RRI data may be denoised by successively applying a series of processing rules in order, where each processing rule is applied over different scales that increase as a size of a sliding analysis window is increased. During the application of each processing rule, maximum and minimum RRI thresholds are selected, and RRIs that exceed the thresholds are marked for removal. As each processing rule is applied, the RRIs marked for removal by previous processing rules are used as context to determine abnormal RRIs at a greater scale. In this way, a standardized procedure for denoising RRI data is described that can be used to increase the quality of the RRI data for tasks such as training models to perform inferences on the RRI data.

## Description

### TECHNICAL FIELD

Embodiments of the subject matter disclosed herein relate to human psychophysiological state detection.

### BACKGROUND

Various technological systems may rely on detection of a user's psychophysiological state to increase the user's performance in various tasks and activities. For example, in the context of driving, stress may affect lateral position and steering metrics, may cause drivers to accelerate and brake more frequently and intensely, and may cause response time and its accuracy to decline. As a result, detecting a driver's psychophysiological state may help improve assistance systems and interventions aimed at sustaining performance and preventing risks on road. Similarly, detecting human states in other types of daily activities at work and in other environments may promote general wellbeing and healthy lifestyles.

A person's psychophysiological state can be detected or estimated from physiological biosignals transmitted from sensors arranged on a person's body, such as electrocardiograph (ECG) data, which may be inputted into a state detection model that estimates the person's psychophysiological state based on the ECG data. However, ECG recordings may contain artifacts that originate from internal (e.g., ectopic beats and movement) and external (e.g., noise and sensor contacts) factors, which may distort physiological dynamics in the ECG data.

### SUMMARY

The current disclosure at least partially addresses one or more of the above identified issues by a method for a computational system for denoising physiological cardiac data, the method comprising extracting R-R interval (RRI) data from the physiological cardiac data; selecting a set of processing rules to apply to the RRI data; selecting a maximum size of a sliding analysis window over which the processing rules are applied to the RRI data; and successively applying the set of processing rules to the RRI data in order, each processing rule applied over different scales that increase as a size of a sliding analysis window is increased, and during the application of each processing rule, marking RRIs that exceed maximum and minimum RRI thresholds as abnormal; removing the RRIs marked as abnormal from the RRI data; and storing the denoised RRI data in a memory of the computational system.

The above advantages and other advantages, and features of the present description will be readily apparent from the following Detailed Description when taken alone or in connection with the accompanying drawings. It should be understood that the summary above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

Various aspects of this disclosure may be better understood upon reading the following detailed description and upon reference to the drawings in which:
FIG. 1 shows a block diagram of an exemplary embodiment of a state detection system configured to assess a stress of a human subject;
FIG. 2 shows an exemplary training system for training a state detection model of the state detection system;
FIG. 3 is a graph of exemplary HR time series data, as prior art;
FIG. 4 shows a flowchart illustrating an exemplary high-level method for preprocessing R-R interval (RRI) data extracted from HR time series data;
FIG. 5 shows a flowchart illustrating a first exemplary method for preprocessing an individual RRI sequence of the RRI data;
FIG. 6 shows a flowchart illustrating an exemplary method for preprocessing RRI data acquired from a plurality of drivers of vehicles for training a state detection model; and
FIG. 7 is a diagram illustrating how values of a data array may be analyzed using sliding analysis windows.

The drawings illustrate specific aspects of the described systems and methods. Together with the following description, the drawings demonstrate and explain the structures, methods, and principles described herein. In the drawings, the size of components may be exaggerated or otherwise modified for clarity. Well-known structures, materials, or operations are not shown or described in detail to avoid obscuring aspects of the described components, systems, and methods.

### DETAILED DESCRIPTION

Methods and systems are described for preprocessing cardiac psychophysiological data of a human subject for detecting a psychophysiological state of the subject. In some examples, the subject may be a user of a technological system, such as a driver of a vehicle, pilot, an operator of a system control panel, etc. In other examples, wearable sensors may be used in the detection and management of psychophysiological states for health purposes. The global wearable device market is a rapidly-growing market as wearable technology becomes an integral part of the Internet of Things in which a network of physical objects/things embedded with electronics, software, sensors, and network connectivity enables these objects/things to communicate with one another and collect and exchange data with one another.

In one example, a person's psychophysiological state may be detected from heart rate (HR) and heart rate variability (HRV) data extracted from psychophysiological data acquired via electrocardiograph (ECG) sensors. In some examples, the HR and/or HRV data may be represented by R-R interval (RRI) data extracted from the psychophysiological data, which represents time intervals in milliseconds between consecutive R peaks of the QRS complex captured by the ECG sensors. The RRI data may be inputted into a state detection model, which may output a prediction or estimation of the person's psychophysiological state based on the RRI data. The state detection model may be a rules-based model, a machine learning (ML) model, or a different type of model.

However, ECG recordings may contain artifacts that originate from internal (e.g., ectopic beats and movement) and external (e.g., noise and sensor contacts) factors, which may distort physiological dynamics in RRI data. To reduce or remove the artifacts, the RRI data may be preprocessed to be denoised. RRI data preprocessing typically involves basic filtering, removal of abnormal beats, interpolation, etc. However, there is no standard approach for denoising RRI data, and typical procedures may lead to loss and distortion of the data. Thus, there is a demand for an RRI data preprocessing model that would be optimal for the calculation of HR and HRV metrics used in psychophysiological state detection.

To address this problem, a robust, standardized method is proposed for preprocessing RRI data that may reduce or remove artifacts. RRI data that has been preprocessed using the method can then be used to train state detection (and other) models to detect psychophysiological states. As a result of training the state detection models on the preprocessed RRI data, a performance and accuracy of the state detection models may be greater than in an alternative scenario where the state detection models are trained on RRI data that is not denoised, or that is denoised using a different procedure.

The method includes applying a combination of thresholds, that may be absolute, differential, and/or relative, within sliding windows of RRI data of varied lengths, to identify abnormal RRIs. A set of simple rules determined by physiological principles may be applied to remove clear artifacts and outliers. Successively more complex rules may be added at each subsequent step, to perform a series of data checks that enhance the quality of the final RRI data. The process may start with small windows of analysis, e.g., checking individual RRIs, the duration of which may increase as more rules are added. The longer the window size, the more RRI data may be potentially identified as abnormal. Any identified abnormal RRIs are marked. Abnormal RRIs as well as short sequences of remaining RRIs surrounded by abnormal data points may be excluded from the subsequent analyses, thereby preparing the data for further checks.

Algorithms designed based on the described method may vary in how many rules are applied, what each rule is testing and what window size and step are selected. The choice of the specific rules, thresholds and window length may be determined by the specifics of data and purposes of each study or application, which may be assessed prior to or at an early stage of processing. That is, an initial assessment may be performed, and based on the initial assessment, the specific rules, thresholds, and window length may be selected, for example, from a lookup table stored in a database. An additional advantage to the method described herein is that it may facilitate tracking back to determine a definite reason why one or more specific RRIs were considered abnormal during the preprocessing.

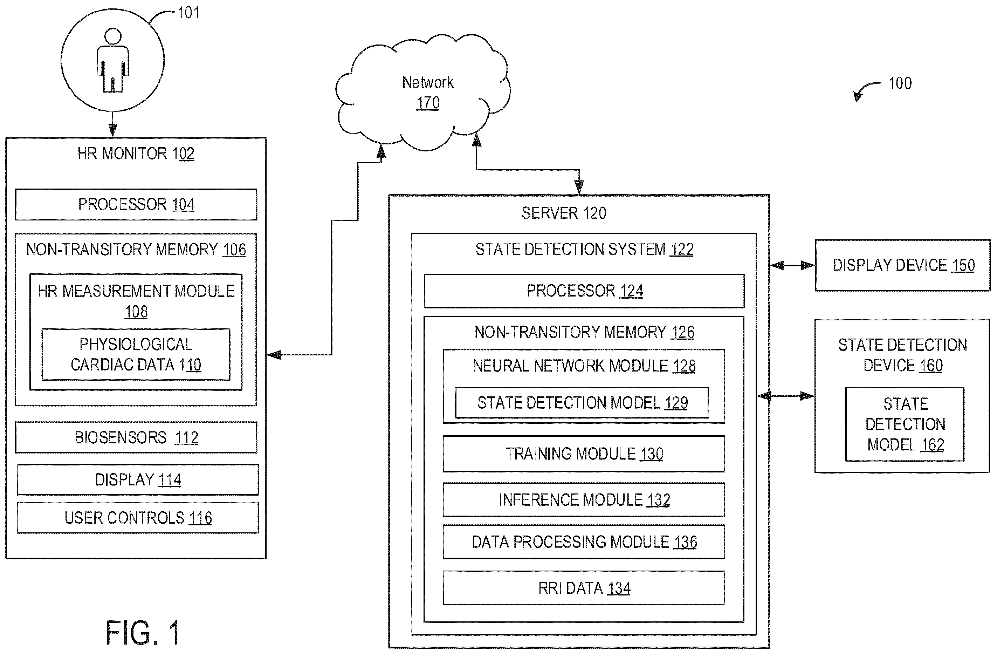

Referring now to FIG. 1, an exemplary state detection system 100 is shown for detecting a psychophysiological state of a human subject 101. State detection system 100 comprises a heart rate (HR) monitor 102, that is communicatively coupled to a state detection system 122 of a server 120, which in various embodiments is a cloud-based server. HR monitor 102 may transmit data, such as cardiac data, to state detection system 122 via a network 170.

HR monitor 102 includes a processor 104 configured to execute machine readable instructions stored in non-transitory memory 106. Processor 104 may be single core or multi-core, and the programs executed thereon may be configured for parallel or distributed processing. In some embodiments, processor 104 may optionally include individual components that are distributed throughout two or more devices, which may be remotely located and/or configured for coordinated processing. In some embodiments, one or more aspects of processor 104 may be virtualized and executed by remotely-accessible networked computing devices configured in a cloud computing configuration.

In some embodiments, HR monitor 102 may be a wearable device worn by the subject. For example, HR monitor 102 may be worn on a wrist of the subject, and/or may be integrated into a device such as a smart watch typically worn on the wrist. In other embodiments, HR monitor 102 may be worn at a different location on a body of the subject. In still other embodiments, one or more portions of HR monitor 102 may be worn on a body of the subject, and other portions of HR monitor 102 may not be worn on a body of the subject, where HR monitor 102 may be in wireless communication with a second device worn on the body of the subject, and the second device may transmit data collected from the subject via the second device (e.g., heart rate, etc.) to the HR monitor 102. Further, in some embodiments, HR monitor 102 may be installed at a vehicle, where subject 101 is a driver of the vehicle.

HR monitor 102 includes one or more biosensors 112. The one or more biosensors 112 may be specially designed devices for sensing a certain type or types of psychophysiological data via placement on a body of subject 101. The one or more biosensors 112 may further include a plurality of sensors of different types or the same type. The one or more biosensors 112 may include sensors for obtaining physiological cardiac data from a subject.

HR monitor 102 may be worn by the subject such that the one or more biosensors 112 are in direct contact with a skin of the subject. For example, HR monitor 102 may be worn on the wrist of the subject, and the one or more biosensors 112 may be positioned on HR monitor 102 such that the one or more biosensors 112 are in face-sharing contact with skin on the wrist. Alternatively, in automotive embodiments, the one or more biosensors 112 may be positioned on a surface of a steering wheel of a vehicle, for example.

The one or more biosensors 112 may include a combination of one or more different kinds of sensor. In various examples, the one or more biosensors 112 include electrocardiograph (ECG) sensors that measure an HR of the subject. The one or more biosensors 112 may also include a photoplethysmographic (PPG) sensor that acquires an optical signal by illuminating skin and detecting changes in absorbed or reflected light with a photodetector. In some embodiments, the one or more biosensors 112 include a remote PPG (rPPG) sensor that senses blood flow in images of the subject acquired via a camera. In various embodiments, the one or more biosensors 112 measure PPG signals at the wrist of subject 101. Changes in the absorbed or reflected light are detected by a photodetector and used to calculate heart activity-related metrics, such as HR and HR variability. Such physiological cardiac data may be used to estimate a state of subject 101. The collected physiological cardiac data may include time-series data collected continuously from subject 101 in real time. It will be appreciated that the types of biosensors listed above are mentioned for illustrative purposes, and the one or more biosensors 112 may additionally include other types of sensors for obtaining physiological cardiac data of a user without departing from the scope of this disclosure.

Physiological cardiac data 110 may be acquired via the one or more biosensors 112, which may include, for example, ECG data, PPG data and/or other physiological cardiac data. Thus, the data sensed by the one or more biosensors 112 correlates to the type of sensors in one or more biosensors 112.

Non-transitory memory 106 may store a heart rate measurement module 108, which may store various programs for measuring and collecting physiological cardiac data 110 via the one or more biosensors 112.

HR monitor 102 further includes a set of user controls 116 and a display 114. User controls 116 may comprise one or more of a touchscreen, a keyboard, a mouse, a trackpad, a motion sensing camera, or other device configured to enable a user to interact with and manipulate data within HR monitor 102. Display 114 may include one or more display devices or screens utilizing virtually any type of technology. In some examples, display 114 may be combined with processor 104, non-transitory memory 106, and/or user controls 116 in a shared enclosure, such as a wristwatch device. In other examples, display 114 may be a peripheral display device and may comprise a monitor, touchscreen, projector, or other display device known in the art, which may enable a user to view an output of HR monitor 102, and/or interact with various data stored in non-transitory memory 106.

In some examples, the one or more biosensors 112 may be external to HR monitor 102, where physiological cardiac data may be transmitted from the one or more biosensors 112 to HR monitor 102 via a wired or wireless connection. For example, HR monitor 102 may be integrated into a vehicle driven by subject 101, and the one or more biosensors 112 may be integrated into components of the vehicle.

Network 170 may include in a non-limiting manner, a wide area network (WAN); a local area network (LAN); the Internet; a wired or wireless (e.g. optical, Bluetooth, Bluetooth Low Energy (BLE), radio frequency (RF) network; a cloud-based computer infrastructure of computers, routers, servers, gateways, etc., or any combination thereof associated therewith that allows one or more computing devices within state detection system 100 to connect with each other. Network 170 may include a public network, or a private network associated with a department of a hospital, for example. In some embodiments, a wireless personal area network (PAN) technology such as MBAN may be used, or induction wireless, infrared wireless, ultra wideband (UWB), Bluetooth^{®}, or any other similar technology for wireless communication between co-located devices.

Server 120 may include a state detection system 122, which may process physiological cardiac data 110 acquired via HR monitor 102. physiological cardiac data 110 may include a plurality of data values generated by HR monitor 102. The data values may be measured by HR monitor 102 at periodic intervals (e.g., once per second, etc.), and transmitted to state detection system 122 as a continuous stream of time-series data. Physiological cardiac data 110 may be streamed wirelessly to state detection system 122 via network 170. Physiological cardiac data 110 may be processed at state detection system 122. In some examples, results of the processing may be transmitted back to HR monitor 102 via network 170.

State detection system 122 includes a processor 124 configured to execute machine readable instructions stored in non-transitory memory 126. Processor 124 may be single core or multi-core, and the programs executed thereon may be configured for parallel or distributed processing. In some embodiments, the processor 124 may optionally include individual components that are distributed throughout two or more devices, which may be remotely located and/or configured for coordinated processing.

Non-transitory memory 126 may store a neural network module 128, a training module 130, an inference module 132, a data processing module 136, and stored RRI data 134 generated by data processing module 136 from physiological cardiac data 110 transmitted from HR monitor 102, as described in greater detail below. Neural network module 128 may include one or more AI models, and may further include various data, or metadata pertaining to the one or more AI models stored therein. In particular, neural network module 128 may include a state detection model 129 for detecting a psychophysiological state of a subject from data received from HR monitor 102 and/or stored in RRI data 134. State detection model 129 may include one or more machine learning models, such as deep learning networks (e.g., convolutional neural networks and/or long short-term memory (LSTM) recurrent neural networks), including a plurality of weights and biases, activation functions, loss functions, gradient descent algorithms, and instructions for implementing the one or more deep neural networks to process the physiological cardiac data.

State detection model 129 may be trained using training data generated from the physiological cardiac data, as described below. The training data may be labeled by experts, in some examples. State detection model 129 may include a classification network or a regression network, which may each include a convolutional layer and/or an LSTM layer. Neural network module 128 may include trained and/or untrained neural networks and may further include training routines, or parameters (e.g., weights and biases), associated with one or more neural network models stored therein.

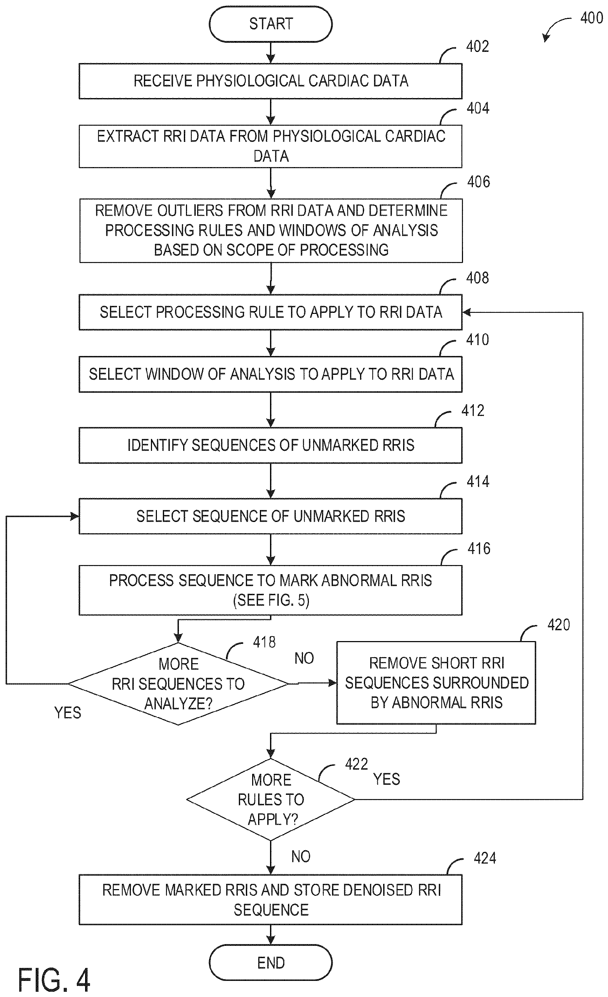

Training module 130 may comprise instructions for training one or more of the neural networks stored in neural network module 128, including state detection model 129. In particular, training module 130 may include instructions that, when executed by the processor 124, cause processor 124 to conduct one or more of the steps of method 400 for training the state detection model, discussed in more detail below in reference to FIG. 4. In some embodiments, training module 130 includes instructions for implementing one or more gradient descent algorithms, applying one or more loss functions, and/or training routines, for use in adjusting parameters of the one or more neural networks of neural network module 128.

Inference module 132 may comprise instructions for implementing a trained state detection model 129 to process new physiological cardiac data received from HR monitor 102. State detection system 122 may be communicatively coupled to a remote state detection device 160, which may be used to detect the psychophysiological state of a user of state detection device 160. The instructions for implementing the trained state detection model to process physiological cardiac data may include instructions for deploying the trained state detection model at state detection device 160. For example, state detection device 160 may be a device worn by a user, or state detection device 160 may be integrated into a vehicle or different environment having controls manipulated by the user. State detection device 160 may detect the psychophysiological state using a state detection model 162, which may be a copy of the trained state detection model 129 stored in inference module 132 that is deployed at state detection device 160. In some embodiments, HR monitor 102 may be included in state detection device 160, or state detection device 160 may include HR monitor 102.

State detection system 122 may include a data processing module 136, which may store instructions for processing physiological cardiac data 110 received from HR monitor 102. In particular, data processing module 136 may extract R-R interval (RRI) data 134 from physiological cardiac data 110, which may be stored in non-transitory memory 126. RRI data 134 may include a continuous sequence of consecutive RRIs calculated from physiological cardiac data 110, where each RRI is a duration between a first R peak of a first QRS complex of physiological cardiac data 110 and a subsequent R peak of a subsequent QRS complex of physiological cardiac data 110. RRI data 134 may capture patterns in physiological cardiac data 110 that may be used to detect the psychophysiological state of a user of state detection device 160, for example. By reformatting physiological cardiac data 110 into RRI data 134, an amount of processing and memory resources of state detection system (e.g., processor 124 and non-transitory memory 126) consumed during training of state detection model 129 may be reduced.

Additionally, data processing module 136 may process the extracted RRI data 134 to remove artifacts (e.g., abnormal RRIs in the RRI data 134). In particular, data processing module 136 may include instructions that, when executed by the processor 124, cause processor 124 to conduct one or more of the steps of methods 400, 500, 600, and 700 of FIGS. 4, 5, 6, and 7, respectively. By removing the artifacts in RRI data 134 (e.g., denoising RRI data 134), a time taken to train state detection model 129 and the amount of processing and memory resources of state detection system consumed during training may be further reduced. Additionally, a performance of the trained state detection model 129 may be increased by denoising RRI data 134 in accordance with methods 400, 500, 600, and/or 700 prior to training.

In some embodiments, data processing module 136 may be independent and external to state detection system 122. For example, data processing module 136 may be included in a separate data processing system installed on server 120, or on a different server communicatively coupled to server 120. In such examples, physiological cardiac data 110 may be transmitted from HR monitor 102 to data processing module 136, processed at data processing module 136, and subsequently transmitted from data processing module 136 to state detection system 122 as RRI data 134. Alternatively, state detection system 122 may transmit physiological cardiac data 110 received from HR monitor 102 to data processing module 136, and may receive processed RRI data 134 from data processing module 136.

Server 120 may be communicatively coupled to a display device 150, such as a computer, where one or more users of state detection system may view and interact with components of state detection system 122. In some examples, display device 150 may include a user interface (UI), which human experts may use to label ground truth data used to train state detection model 129.

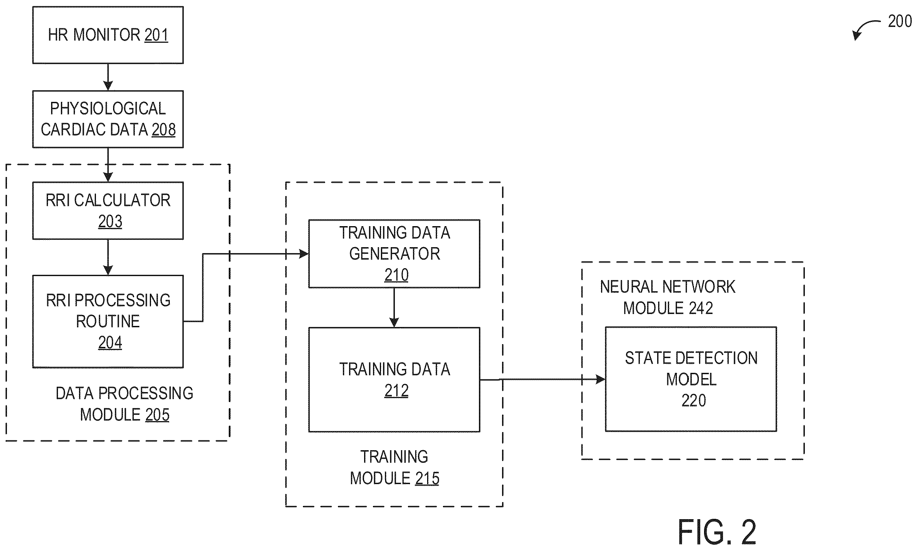

Turning now to FIG. 2, an exemplary state detection model training system 200 is shown, which may be used to train a state detection model 220 (e.g., state detection model 129 of FIG. 1). State detection model 220 may be trained to detect a psychophysiological state of a human subject from physiological cardiac data 208 acquired from the human subject via a HR monitor 201 (e.g., HR monitor 102).

Physiological cardiac data 208 may include a continuous stream of cardiac parameters acquired in real time by one or more biosensors (e.g., biosensors 112). State detection model 220 may be stored within a neural network module 242 of the state detection system. Neural network module 242 may be a non-limiting example of neural network module 128 of state detection system 100 of FIG. 1.

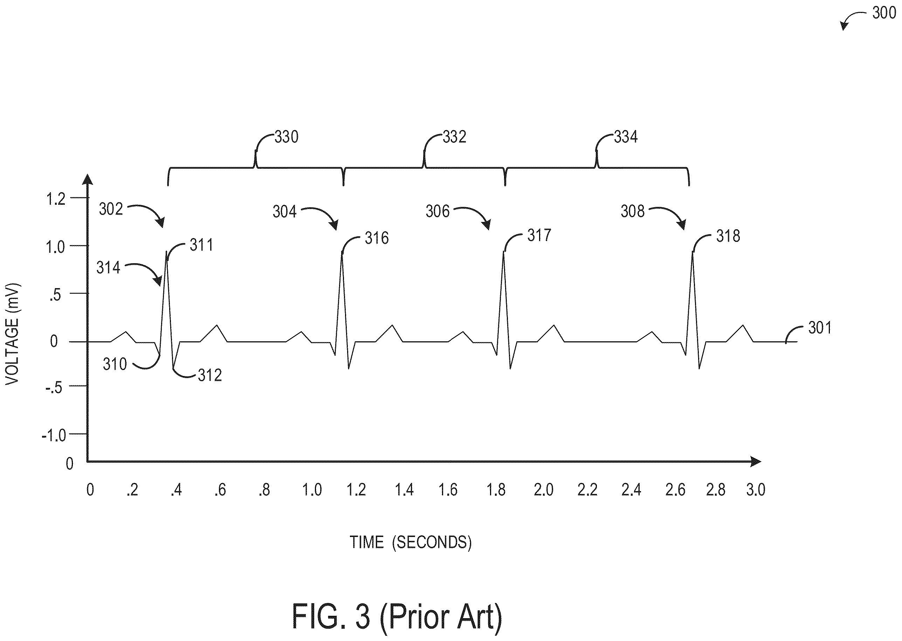

State detection model training system 200 may include an RRI calculator 203, which may calculate RRI data from physiological cardiac data 208. RRI calculator 203 may be stored within a data processing module 205 of the state detection system. Data processing module 205 may be a non-limiting example of data processing module 136 of state detection system 100 of FIG. 1. RRI calculator 203 may take the cardiac parameters of physiological cardiac data 208 as input, and may extract an RRI data stream from the physiological cardiac data 208. The calculation of RRIs from physiological cardiac data 208 is described in greater detail below in reference to FIG. 3.

The RRI data steam may additionally be processed by an RRI processing routine 204 of data processing module 205. RRI processing routine 204 may reduce or remove artifacts from (e.g., denoise) the RRI data stream prior to training state detection model 220. RRI processing routine 204 may implement one or more of methods 400, 500, 600, and 700 described below in reference to FIGS. 4, 5, 6, and 7, respectively.

Training module 215 may include a training data generator 210, which may generate training data 212 used to train state detection model 220. Training data 212 may comprise labelled RRI data. State detection model 220 may be trained on training data 212 to learn to detect one or more psychophysiological states of new subjects, based on new physiological cardiac data acquired via HR monitor 201, using various techniques known in the art. In some embodiments, state detection model 220 may alternatively be a rule-based model generated from training data 212, rather than an ML model. In such embodiments, the training dataset may be used to generate the rule-based model.

FIG. 3 shows a time-series graph 300 of physiological cardiac data of a subject, such as physiological cardiac data 208 of training system 200 of FIG. 2, depicted as a plot 301. The HR may be detected by a monitoring device, such as HR monitor 102 and/or HR monitor 201.

Plot 301 shows various cardiac cycles of the physiological cardiac data, where each cardiac cycle of the various cardiac cycles begins and ends with a heartbeat. A first cardiac cycle 330 begins with a first heartbeat 302 and ends with a second heartbeat 304; a second cardiac cycle 332 begins with second heartbeat 304 and ends with a third heartbeat 306; and a third cardiac cycle 334 begins with third heartbeat 306 and ends with a fourth heartbeat 308. Each heartbeat is characterized by an increase in blood pressure as the heart contracts to push blood out to a body of the patient during a systolic portion of the cardiac cycle, and a subsequent decrease in blood pressure as the heart relaxes to fill with blood received from veins of the body during a diastolic portion of the cardiac cycle.

Each heartbeat may be represented by a combination of three waves of the physiological cardiac data during the depolarization of ventricles of a heart of the subject and a contraction of ventricular muscles of the subject. The three waves include a Q wave 310 (e.g., a negative deflection of the physiological cardiac data from a baseline), an R wave 311 (a positive deflection of the physiological cardiac data from the baseline) also referred to as R-Peak 311, and an S wave 312 (a negative deflection of the physiological cardiac data from the baseline). Together, Q wave 310, R wave 311, and S wave 312 may be referred to as a QRS complex 314.

For a subject with a normal heart, each cardiac cycle may have a slightly different duration, due to normal variability in the HR of the patient. For example, first cardiac cycle 330 may have a first duration, measured between R-peak 311 and a subsequent R-peak 316; second cardiac cycle 332 may have a second duration, measured between R-peak 316 and a subsequent R-peak 317, where the second duration is shorter or longer than the first duration; and third cardiac cycle 334 may have a third duration, measured between R-peak 317 and a subsequent R-peak 318, where the third duration may be shorter or longer than either or both of the first duration and second duration. In the depicted example, second cardiac cycle 332 has a slightly shorter duration than first cardiac cycle 330, and third cardiac cycle 334 has a slightly longer duration than first cardiac cycle 330.

The physiological cardiac data depicted in plot 301 may converted from a stream of continuous values (e.g., voltages) to a corresponding stream of RRI values, where each RRI value is a duration between consecutive R-peaks of the physiological cardiac data. For example, a first RRI value may correspond to a duration of first cardiac cycle 330, starting at R-peak 311 and ending at R-peak 316 (0.8 seconds); a second RRI value may correspond to a duration of second cardiac cycle 332, starting at R-peak 316 and ending at R-peak 317; a third RRI value may correspond to a duration of third cardiac cycle 334, starting at R-peak 317 and ending at R-peak 318; and so on. Thus, plot 301 may be represented by an RRI sequence [0.8, 0.7, 1.0]. In this way, the physiological cardiac data may be converted to a simpler representation, which may facilitate further analysis. For example, detecting a psychophysiological state of the subject may be based on HR and HRV indices calculated from the RRI sequence.

Referring now to FIG. 4, a flowchart is shown of a method 400 for processing and denoising RRI data acquired from an HR monitor, such as HR monitor 102 of FIG. 1 and/or HR monitor 201 of FIG. 2. In some examples, the processed RRI data may be used for training a state detection model, such as state detection model 129 of FIG. 1 and/or state detection model 220 of FIG. 2. In such examples, method 400 and the other methods described herein may be executed by a processor of a state detection system, such as state detection system 122 of FIG. 1. Operations of method 400 and the other methods described herein may be stored in non-transitory memory of the state detection system (e.g., in non-transitory memory 126) and executed by a processor of the state detection system (e.g., processor 124). In other examples, the processed RRI data may be used for a different purpose, and method 400 and the other methods described herein may be executed by a processor of a different system. For example, in some embodiments, method 400 may be executed by a processor of a state detection device, such as state detection device 160 of FIG. 1.

Method 400 begins at 402, where method 400 includes receiving physiological cardiac data from one or more human subjects, via biosensors (e.g., biosensors 112) arranged on the one or more human subjects. The physiological cardiac data may include data that may be acquired, for example, via a PPG sensor or an ECG sensor of the biosensors. The biosensors may be in contact with a skin of the subject. For example, the one or more human subjects may wear the HR monitor, a wristwatch, or other lifestyle devices containing sensors during normal daily activities. In other applications, the biosensors may be integrated into one or more devices used by the one or more human subjects, such as a steering wheel of a vehicle.

In various embodiments, the physiological cardiac data is collected from a plurality of human subjects representing a diverse population, where the human subjects include subjects of different ages, genders, ethnicities, backgrounds, etc. By collecting the physiological cardiac data from the diverse population, inferences made, for example, by a model, trained on the physiological cardiac data may be more accurate when applied to subjects of a general population that is similarly diverse. However, a disadvantage of collecting data from the plurality of human subjects representing a diverse population is that a variability of HR (RRI) data extracted from the physiological cardiac data may be greater, where a greater amount of abnormal data may be received. As a result, the HR data extracted from the physiological cardiac data may undergo processing to remove artifacts from the physiological cardiac data, as described in the steps below.

At 404, method 400 includes extracting an RRI data array from the physiological cardiac data. As described above, the RRI data may include sequences of consecutive RRIs calculated from the acquired physiological cardiac data. The RRI data captures heart beat variability data in a simpler representation as the physiological cardiac data, as described above in reference to FIG. 3.

At 406, method 400 includes performing initial processing of the RRI data to remove outliers. Absolute maximum and minimum thresholds for RRI values may be selected, for example, from a lookup table stored in a memory of the state detection system (e.g., non-transitory memory 126). Each individual RRI of the RRI data may be compared to the maximum and minimum thresholds, and RRI values that are greater than the maximum threshold or less than the minimum threshold may be marked as abnormal. The number of RRIs marked as abnormal may then be evaluated, to determine a scope of processing to be performed on the RRI data. Specifically, a set of processing rules to apply to the RRI data may be selected based on the number of abnormal RRIs. Additionally, a maximum size of a sliding window of analysis used to assess the RRI data may be selected based on the number of abnormal RRIs and the purposes of subsequent data processing.

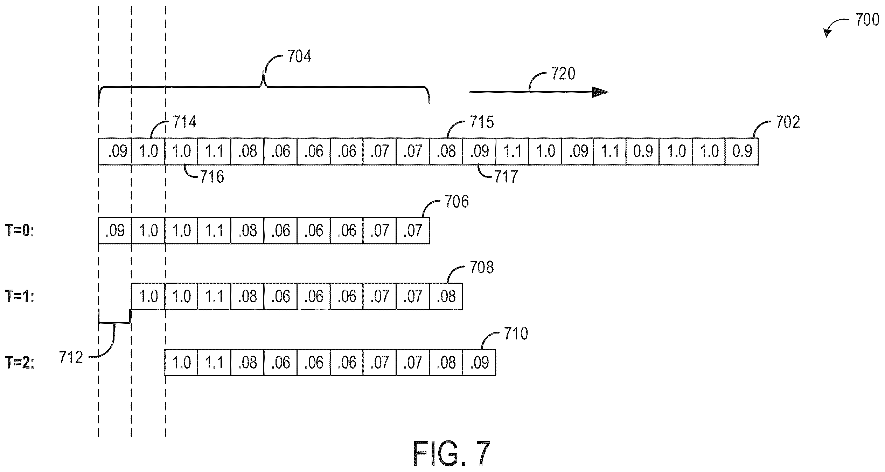

Referring briefly to FIG. 7, a diagram 700 shows how an RRI data array 702 may be processed using a sliding window 704, in accordance with an embodiment of method 400. Sliding window 704 comprises a subset of a total amount of RRI values included in RRI data array 702. In the depicted example, sliding window 704 includes 10 RRI values. In other embodiments, sliding window 704 may comprise a different number of RRI values.

Sliding window 704 may be configured to advance along RRI data array 702 in time increments in a direction indicated by an arrow 720 as RRI data array 702 is processed (e.g., by method 400). Thus, at a first time increment T=0, sliding window 704 includes a first array 706 comprising the first 10 RRI values of RRI data array 702. At a second time increment T=1, sliding window 704 is moved in the direction of arrow 720 by a step size 712. In the depicted example, step size 712 is one RRI value; in other embodiments, step size 712 may be a different number of RRI values, such as 5, or 10 RRI values, depending on a desired overlap. As a result of moving by step size 712, at time increment T=1, sliding window 704 includes a second data array 708 comprising 10 RRI values of RRI data array 702 starting with a second RRI value 714 of RRI data array 702, and ending with an eleventh RRI value 715 of data array 702. At a third time increment T=3, sliding window 704 is once again moved in the direction of arrow 720 by step size 712. As a result of moving by step size 712, at time increment T=3, sliding window 704 includes a third data array 710 comprising 10 RRI values of RRI data array 702, starting with a third RRI value 716 of RRI data array 702, and ending with a twelfth RRI value 717 of data array 702. RRI data array 702 may be analyzed in this manner until sliding window 704 reaches an end of RRI data array 702, with a last data array comprising the last 10 values of RRI data array 702.

In this way, RRI data array 702 may be analyzed for abnormal RRI values (e.g., above or below a threshold) dynamically by one or more processing rules within a context of a predetermined number of RRI values. By adjusting a size (e.g., length, duration in time, or number of RRI values) of sliding window 704, abnormalities in RRI values may be detected at different scales.

For example, if sliding window 704 includes two RRI values, a target RRI value may be detected as abnormal based on a difference between the target RRI value and a second RRI value of sliding window 704 (e.g., a large difference between the target RRI value and the second RRI value may indicate abnormality at a small scale). If sliding window 704 includes 10 RRI values, a target RRI value may be detected as abnormal based on a difference between the target RRI value and a group of 10 consecutive RRI values of sliding window 704 (e.g., a difference between the target RRI value and a mean RRI value of the 10 RRI values may indicate abnormality at a larger scale). If sliding window 704 includes 20 RRI values, a target RRI value may be detected as abnormal based on a difference of the target RRI value with respect to a group of 20 consecutive RRI values of sliding window 704 (e.g., a difference between the target RRI value and a mean RRI value of the 20 RRI values may indicate abnormality at an even larger scale). Thus, by processing RRI data array 702 iteratively using sliding windows 704 that increase over time, abnormal RRI values may be detected at various scales, where some of the abnormal RRI values would not be detected if RRI data array is analyzed using sliding windows of a single size.

Returning to method 400, the set of processing rules and maximum size of the window of analysis may be determined based on specifics of the physiological cardiac data and purposes of a relevant study or application. In various examples, set of processing rules and maximum size of the window of analysis may be established by human experts based on empirical evidence and retrieved from a lookup table stored in the memory of the state detection system.

Steps 408-422 may be performed in an iterative or cyclical manner, where the set of processing rules are performed in order. Each processing rule may be applied over different scales that increase as a size of a selected sliding analysis window is increased, starting with a smallest analysis window (e.g., an individual RRI value) and ending with the maximum sized window of analysis, as described in reference to FIG. 7. During the application of each processing rule, maximum and minimum RRI thresholds may be selected, which may be different for each processing rule and/or sliding window size. RRIs that are above or below the maximum and minimum thresholds respectively are marked for removal. In this way, as each processing rule is applied, the RRIs marked for removal by previous processing rules are used as context to determine abnormal RRIs at a greater scale.

At 408, method 400 includes selecting a processing rule of the set of processing rules to apply to the RRI data, and at 410, method 400 includes selecting a window of analysis to apply to the RRI data.

At 412, method 400 includes identifying a plurality of sequences of unmarked RRIs. In other words, at each iteration of method 400, various RRIs may be marked as abnormal, leaving sequences of consecutive unmarked RRIs between the marked RRIs. Each sequence of unmarked RRIs may be processed iteratively in accordance with steps 414-422.

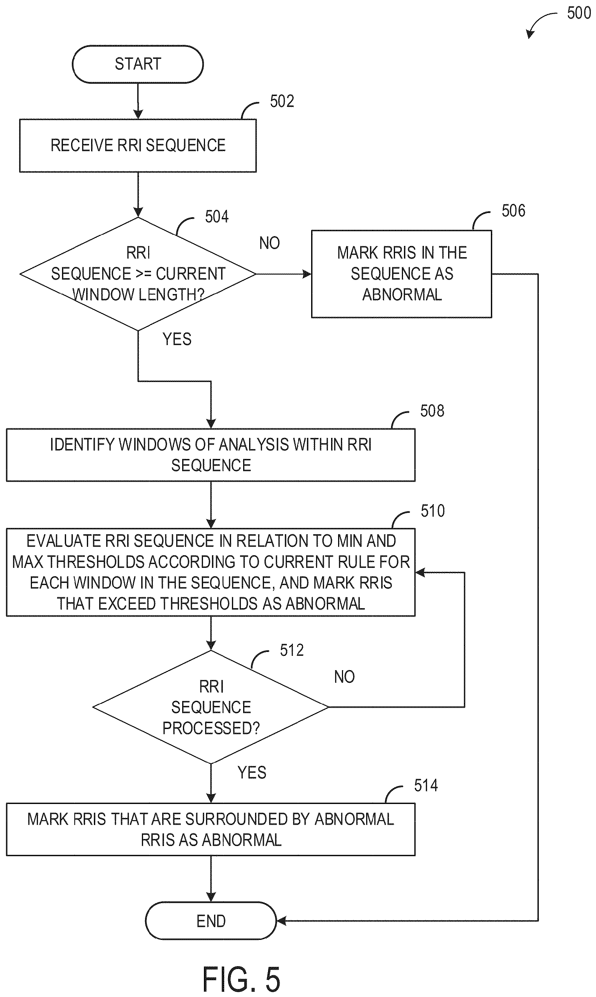

At 414, method 400 includes selecting an RRI sequence of the unmarked RRIs, and at 416, method 400 includes processing the RRI sequence to mark abnormal RRIs in the selected RRI sequence. Processing the RRI sequence is described below in reference to FIG. 5.

At 418, method 400 includes determining whether there are more RRI sequences of the plurality of RRI sequences to analyze. If there are more RRI sequences to analyze, method 400 proceeds back to 414, and a next RRI sequence of the plurality of sequences of unmarked RRIs is selected for processing in accordance with step 416. Alternatively, if at 418 there are no more RRI sequences to analyze, method 400 proceeds to 420.

At 420, method 400 includes removing short RRI sequences surrounded by abnormal RRIs from the RRI data prior to performing a subsequent rule. That is, prior to performing the subsequent rule, another check is performed for normal RRI sequences that are less than a threshold sliding analysis window length and surrounded by abnormal RRIs. The short RRI sequences may then be removed.

At 422, method 400 includes determining whether there are more processing rules to apply to the sequences of unmarked RRIs. If at 422 there are more processing rules to apply to the sequences of unmarked RRIs, method 400 proceeds back to 408, where method 400 includes selecting a subsequent processing rule to apply to the sequences of unmarked RRIs. If at 422 it is determined that there are no more processing rules to apply, method 400 proceeds to 424.

At 424, method 400 includes removing the RRIs marked as abnormal from the RRI data, and outputting a clean RRI sequence, where the clean RRI sequence comprises RRIs that are not marked as abnormal as a result of the processing of the RRI data by the processing rules. That is, the RRIs that are marked as abnormal are eliminated from the RRI data, leaving RRIs that are determined to be within the defined thresholds applied for each processing rule.

Referring now to FIG. 5, a method 500 is shown for processing an RRI sequence comprising a plurality of consecutive RRIs that have not been marked as abnormal, of a larger set of RRI data processed in accordance with method 400 of FIG. 4. Thus, method 500 may be performed as part of method 400.

Method 500 starts at 502, where method 500 includes receiving the RRI sequence. At 504, method 500 includes determining whether the RRI sequence is greater than or equal to a length of a currently selected window of analysis. If at 504 it is determined that the RRI sequence is not greater than or equal to the length of the currently selected window of analysis, method 500 proceeds to 506, where method 400 includes marking the RRI sequence as abnormal. Marking the RRI sequence as abnormal marking may comprise marking every RRI value in the RRI sequence as abnormal. That is, short RRI sequences may be considered abnormal and eliminated.

Alternatively, if at 504 it is determined that the RRI sequence is greater than or equal to the length of the currently selected window of analysis, method 500 proceeds to 508. At 508, method 500 includes identifying windows of analysis within the RRI sequence. Identifying the windows of analysis includes determining how many different windows of analysis of different sizes are applied to the RRI sequence. In various embodiments, the different windows of analysis applied to the RRI sequence may be predefined based on an initial assessment of the RRI data, and retrieved from a lookup table of the state detection system.

At 510, method 500 includes evaluating the RRI data in relation to minimum and maximum thresholds, in accordance with a current processing rule (e.g., selected at step 408 of method 400), for each window of analysis in the sequence. RRIs that are greater than the maximum threshold or less than the minimum threshold are marked as abnormal. RRIs that are within the minimum and maximum thresholds are not marked as abnormal.

For example, the current processing rule may include comparing each RRI value of the RRI sequence with an average (e.g., mean) value of all other RRIs included in each sliding window applied to the RRI sequence. A first sliding window of analysis of a first size may be selected and applied to the RRI sequence in a first iteration. The first size may comprise a single RRI value of the RRI sequence, where the current processing rule is applied individually to each target RRI of the RRI sequence. During the first iteration, each target RRI value of the RRI sequence may be compared to a first minimum threshold and a first maximum threshold. If a respective target RRI value is greater than the first maximum threshold or less than the first minimum threshold, the target RRI value may be marked as abnormal.

A second sliding window of analysis of a second size may then be selected and applied to the RRI sequence in a second iteration. The second size may comprise, for example, a pair of two consecutive RRI values of the RRI sequence, where the current processing rule is applied to each target RRI value of the RRI sequence with respect to an RRI pair including the target RRI value. During the second iteration, each target RRI value of the RRI sequence may be compared to an average (e.g., mean) RRI value of the two RRI values of the pair including the target RRI value. A second minimum threshold distance and a second maximum threshold distance may be selected for the second sliding window of analysis (e.g., retrieved from a lookup table). If a difference between a respective target RRI value and the average RRI value for a respective pair is greater than the second maximum threshold distance or less than the second minimum threshold distance, the target RRI value may be marked as abnormal. The second sliding window of analysis may be applied in steps of one RRI value, where the second sliding window is moved along the RRI sequence by one RRI value at a time, and the rule is applied each time the sliding window is moved.

A third sliding window of analysis of a third size may then be selected and applied to the RRI sequence in a third iteration. The third size may comprise, for example, 10 consecutive RRI values of the RRI sequence, where the current processing rule is applied to each target RRI value of the RRI sequence with respect to 10 RRI values included in a respective sliding window of analysis including the target RRI value. During the third iteration, each target RRI value of the RRI sequence may be compared to an average RRI value of the 10 RRI values of the sliding window. A third minimum threshold distance and a third maximum threshold distance may be selected for the third sliding window of analysis (e.g., retrieved from a lookup table). If a difference between a respective target RRI value and the average RRI value for the third sliding window is greater than the third maximum threshold distance or less than the third minimum threshold distance, the target RRI value may be marked as abnormal. The third sliding window may be applied in steps of five RRI values, where the third sliding window is moved along the RRI sequence by five RRI values at a time, and the rule is applied each time the sliding window is moved. In other examples, the step size may be different. In various examples, the step size may be predetermined for the size of sliding analysis window and retrieved from a lookup table.

A fourth sliding window of analysis of a fourth size may then be selected and applied to the RRI sequence in a fourth iteration. The fourth size may comprise, for example, 30 seconds of consecutive RRI values of the RRI sequence, where the current processing rule is applied to each target RRI value of the RRI sequence with respect to a number of RRI values included in a respective sliding window of a 30 second duration including the target RRI value. During the fourth iteration, each target RRI value of the RRI sequence may be compared to an average (e.g., mean) RRI value of the number of RRI values of the fourth sliding window. A fourth minimum threshold distance and a fourth maximum threshold distance may be selected for the fourth window of analysis (e.g., retrieved from a lookup table). If a difference between a respective target RRI value and the average RRI value for the sliding window is greater than the fourth maximum threshold distance or less than the fourth minimum threshold distance, the target RRI value may be marked as abnormal. The fourth sliding window may be applied in steps of 10 seconds, for example, where the fourth sliding window is moved along the RRI sequence by a number of RRI values corresponding to 10 seconds at a time, and the rule is applied each time the sliding window is moved. The step size may be predetermined for the size of sliding analysis window and retrieved from a lookup table.

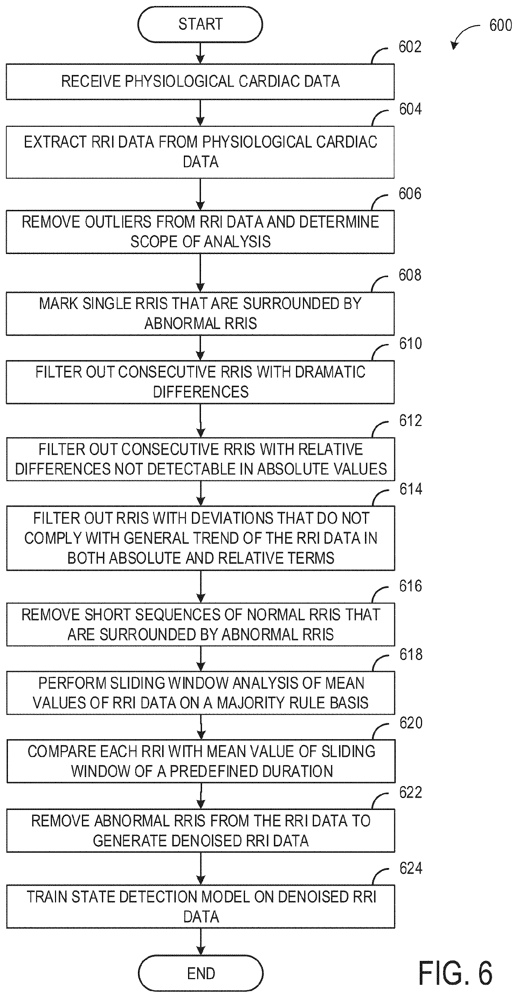

The RRI sequence may be processed as described above until all the sliding windows of the maximum sliding window size have been evaluated. A next processing rule may then be selected, and a similar process may be performed for the next processing rule. As an example, a subsequent processing rule may include a "majority rule" comparison, where rather than marking individual RRIs that exceed respective thresholds as abnormal, all RRIs of a respective sliding window (or RRI sequence) may be marked as abnormal if a predetermined majority (e.g., percentage) of the RRIs exceed the respective thresholds. An example application of the majority rule is described below in reference to FIG. 6.

In this way, a predefined series of processing rules may be iteratively and/or cyclically applied to the RRI data to remove RRIs that are outside of the predefined thresholds. The RRI data evaluation procedures are based on physiological principles that determine different min and max thresholds of possible variation in RRI data series at each step of the analysis. RRIs that exceed at least one threshold are identified and marked as abnormal. Each step of the analysis is run on selected window size, e.g., from a single RRI to sequences of RRIs, or time intervals (e.g., 30 sec) of the RRI data. The window size may be increased at each cycle, where the process starts with running selected rules on shorter windows, and proceeds to apply rules to increasingly longer windows. RRI sequences shorter than the current window of analysis that are surrounded by abnormal RRIs are also marked as abnormal. Thus, each consecutive processing rule is run on RRIs that have not been marked as abnormal in a previous cycle/iteration of the process.

At 512, method 500 includes determining whether the RRI sequence has been processed by all selected sliding window sizes. If the RRI sequence has not been processed by all selected sliding window sizes, method 500 proceeds back to 510, where method 500 includes continuing to evaluate the RRI sequence with the remaining selected sliding window sizes. Alternatively, if the RRI sequence has been processed by all selected sliding window sizes, method 500 proceeds to 514.

At 514, method 500 includes marking RRIs that are surrounded by abnormal RRIs as abnormal. In some cases, single RRIs that are surrounded by abnormal RRIs are marked as abnormal. In other cases, pairs of RRIs that are surrounded by abnormal RRIs may additionally or alternatively be marked as abnormal. In still other cases, longer sequences of RRIs that are surrounded by abnormal RRIs may additionally or alternatively be marked as abnormal. In other words, when unmarked RRIs occurring individually, in pairs, or in sequences are surrounded by abnormal RRIs, it may be inferred that the unmarked RRIs may be abnormal as well. The size of sequence of unmarked RRIs that may be marked as abnormal when surrounded by abnormal RRIs may depend on the size of sliding window. Method 500 ends.

The general principles described above can be used to design a variety of algorithms with different rules and thresholds for RRI data preprocessing. The rules and thresholds would depend on the purposes of use and specifics of the data. As an example of how method 400 may be applied in a specific scenario, FIG. 6 describes a solution for detecting a physiological state of a driver of a vehicle that describes one implementation of method 400.

Referring now to FIG. 6, a flowchart is shown of a method 600 for training a state detection device to be installed at a vehicle that detects physiological states of a variety of different types of drivers, of both genders and different age groups, ethnicities, and backgrounds, from physiological cardiac data collected while the different types of drivers are driving (or driving a simulator), where the data includes RRI data that is preprocessed in accordance with the methods described above. The state detection device may be trained on training data comprising RRI data that is homogenized or standardized across the diverse genders, age groups, ethnicities, and backgrounds. To obtain the homogenized or standardized training data, raw RRI data acquired from a plurality of different drivers may be processed and denoised using a variation of method 400 described above. In some embodiments, method 600 may be applied individually to data of each driver. In other embodiments, method 600 may be applied to RRI data collected and aggregated from the plurality of different drivers.

Method 600 begins at 602, where method 600 includes receiving physiological cardiac data from one or more biosensors of a HR monitor (e.g., biosensors 112 of HR monitor 102) arranged in contact with skin of the plurality of different drivers. The physiological cardiac data may be time-series data received in real time from and stored in a memory (e.g., non-transitory memory 126). For example, the biosensors may be worn by the user, or integrated into a vehicle driven by the user, or arranged in contact with the skin of the user in a different manner. For example, the physiological cardiac data may be acquired, for example, via a PPG sensor or an ECG sensor of the biosensors that is integrated into steering wheels of vehicles driven by the plurality of drivers.

At 604, method 600 includes extracting an RRI data array from the physiological cardiac data. As described above, the RRI data array may include sequences of consecutive RRIs calculated from the acquired physiological cardiac data. The RRI data may capture heart beat variability data, as described above in reference to FIG. 3.

At 606, method 600 includes removing outliers from the RRI data, meaning, filtering out RRIs based on absolute thresholds. A first set of minimum and maximum absolute thresholds may be retrieved from a lookup table and used to identify and mark any single RRIs that are out of an expected physiological range. The first set of minimum and maximum absolute thresholds may be based on recommendations of a cardiology association, such as the European Society of Cardiology, or psychophysiological usage recommendations in relevant literature, depending on the type of tasks in the study or other intended usage. A set of processing rules to apply to the RRI data may then be selected based on the number of abnormal RRIs detected and marked, which are applied in steps 608-622. Additionally, a maximum size of a sliding window of analysis used to assess the RRI data may be selected based on the number of abnormal RRIs. The maximum size of the sliding window of analysis may be established by human experts and may be based on similar recommendations of the cardiology association or psychophysiological usage recommendations. The set of processing rules and maximum size of the window of analysis may be retrieved from a lookup table stored in the memory of the state detection system.

At 608, after the RRIs have been filtered based on the absolute thresholds, any single RRIs surrounded by abnormal RRIs may also be marked as abnormal. It cannot be assumed that these single RRIs in the normal range are not affected by artifacts around them, and such data may not add value to the description of the cardiac dynamics during this interval of time. In various embodiments, step 608 may be repeated after various processing steps.

At 610, method 600 includes applying a first processing rule, which includes filtering out consecutive RRIs with dramatic differences. That is, the algorithm checks for dramatic differences between two consecutive RRIs, which is considered an artifact. The dramatic differences may include dramatic incremental differences (e.g., a dramatic increase in RRI length between a first RRI and a second, subsequent RRI), and dramatic decremental differences (e.g., a dramatic decrease in RRI length between the first RRI and the second, subsequent RRI). Specifically, a threshold difference may be retrieved from a lookup table, and each target RRI of the RRI data may be compared to an immediately preceding RRI of the RRI data. If a difference between the target RRI and the immediately preceding RRI is greater than the threshold difference (in absolute terms), the target RRI may be marked as abnormal. Once the above process has run, any remaining single RRIs surrounded by abnormal RRIs may also be marked as abnormal (step 608).

At 612, method 600 includes applying a second processing rule to the RRI data to filter out consecutive RRIs with relative differences not detectable in a comparison of absolute values. This rule addresses dramatic changes between each target RRI relative to the immediately preceding RRI on a percentage basis. A threshold percentage may be retrieved from a lookup table, and each target RRI of the RRI data may be compared to the immediately preceding RRI. If a relative difference between the target RRI and the immediately preceding RRI is greater than the threshold percentage, the target RRI may be marked as abnormal. For example, the threshold percentage may be 30%, where if the target RRI is at least 30% greater than the immediately preceding RRI, the target RRI may be marked as abnormal. Once the above process has run, any remaining normal single RRIs or pairs of normal RRIs surrounded by abnormal RRIs may also be marked as abnormal.

At 614, method 600 includes applying a third processing rule to the RRI data to filter out RRIs with deviations that do not comply with a general trend of the RRI data. This rule includes evaluating the difference between each target RRI and a mean value of an immediately preceding RRI and a subsequent consecutive RRI. A predefined threshold difference for the third processing rule may be retrieved from a lookup table. If the difference between the target RRI and the mean value is greater than the predefined threshold difference, the RRI may be marked as abnormal. In various embodiments, the third processing rule is performed twice: a first time in which an absolute difference is calculated and compared with an absolute threshold value, and a second time in which a relative difference is calculated and compared with an relative threshold value (e.g., a percentage). Once the third processing rule has been applied, any remaining normal single RRIs or pairs of normal RRIs surrounded by abnormal RRIs may also be marked as abnormal.

At 616, method 600 includes applying a fourth processing rule to mark sequences of normal RRIs that are less than a threshold sequence length and that are surrounded by abnormal RRIs as abnormal. The threshold sequence length may be retrieved from a lookup table based on the fourth processing rule. In various examples, step 616 may additionally be performed after other steps of method 600.

At 618, method 600 includes applying a fifth processing rule to perform a sliding window analysis of mean values, for each target RRI in the RRI data, on a majority rule basis, where the sliding window corresponds to a predefined number of RRIs. In one example, the sliding window includes 10 RRIs, and the RRI data is analyzed by advancing the sliding window through the RRI data 5 RRIs at a time. In other embodiments, a different step size may be used. The predefined number of RRIs and the step size may be retrieved from a lookup table based on the fifth processing rule.

To perform the fifth processing rule, mean values are computed for the RRIs included in each sliding window. A threshold difference and a threshold percentage for the fifth processing rule may be retrieved from a lookup table. Each target RRI within the sliding window is then compared to the mean value. If a target RRI differs from the mean value by more than the threshold difference, the target RRI is marked as abnormal. Then, after all the RRIs in the sliding window have been evaluated, the number of abnormal RRIs in the sliding window is compared to the threshold percentage. If the proportion of abnormal RRIs in the sliding window is greater than the threshold percentage, then all of the RRIs within the window are marked as abnormal. For example, if at least 80% of the RRIs are not within the selected thresholds, then all 10 RRIs within the window may be marked as abnormal. After the RRIs are marked, short sequences of normal RRIs surrounded by marked RRIs may also be marked as abnormal (e.g., step 616).

At 620, method 600 includes applying a sixth processing rule to perform a sliding window analysis of mean values for each target RRI in the RRI data, where the sliding window corresponds to a predefined duration. In one example, the predefined duration is 30 seconds, and the RRI data is analyzed by advancing the sliding window through the RRI data 10 seconds at a time. In other embodiments, a different step size (e.g., duration) may be used for the sixth processing rule. A threshold difference for the sixth processing rule may be retrieved from a lookup table. A mean value of all the RRIs included in each sliding window is then computed, and each target RRI within the sliding window is compared to the mean value. If the difference between the target RRI and the mean value is greater than the threshold difference, the target RRI is marked as abnormal. In various embodiments, the sixth processing rule may be performed twice, once where the threshold difference is an absolute difference, and once where the threshold difference is a relative difference based on a percentage. After the RRIs are marked, short sequences of normal RRIs surrounded by marked RRIs may be removed, as described above.

At 622, after the processing rules of the set of processing rules have been performed, method 600 includes removing the RRIs marked as abnormal during the application of the processing rules. By removing the abnormal RRIs, a denoised RRI data array is generated that includes RRIs that are within expected thresholds, and does not include RRIs that are either greater or smaller than expected.

At 624, method 600 includes training the state detection model on the denoised RRI data. The state detection model may be trained in accordance with techniques known in the art. By training the state detection model on the denoised RRI data rather than the RRI data prior to performing method 600, or RRI data denoised using a different procedure, an accuracy of the state detection model may be increased and an amount of training time and computational resources consumed during training may be advantageously reduced, resulting in an overall increase in efficiency of training the state detection model.

In this way, the maximum and minimum thresholds are dynamically selected in accordance with a current processing rule. The thresholds used for one processing rule may be different from thresholds used for a different processing rule. This allows for the identification of abnormal RRIs in the context of a varying time frames and scales.

Thus, a robust, standardized method is proposed for preprocessing RRI data to reduce or remove artifacts prior to further analysis and/or training of a model, such as a state detection models that detects psychophysiological states. A performance and accuracy of models trained on the preprocessed RRI data may be increased with respect to other approaches. The method includes first selecting a suitable set of processing rules to apply to the RRI data, and then applying the selected processing rules to the RRI data in a specific order. The processing rules iteratively detect abnormal RRIs at different scales in the RRI data and mark them for removal. Global outliers may first be removed. In various embodiments, the processing rules may be selected based on a number of global outliers detected. Additionally, a maximum size of a sliding analysis window may be selected for each processing rule. The processing rules and the maximum sizes of the sliding analysis windows may be selected based on a variability of the RRI data. For example, if a larger number of global outliers are detected, it may be inferred that an amount of variability of the RRI data is higher, whereby a first set of processing rules and maximum sliding window sizes may be selected. Alternatively, if a smaller number of global outliers are detected, it may be inferred that the amount of variability of the RRI data is lower, whereby a second set of processing rules and maximum sliding window sizes may be selected. The second set of processing rules may be smaller than the first set, and the maximum size of the sliding windows used in the second set of processing rules may be larger.

In other examples, the processing rules and the maximum sizes of the sliding analysis windows may be selected to increase a computational efficiency of a model training system. That is, if the amount of variability of the RRI data is inferred to be higher based on the detection of a larger number of global outliers, it may also be inferred that an amount of processing performed to denoise the RRI data may be increased. As a result, a smaller number of processing rules may be used, and/or smaller analysis windows, to reduce an overall amount of processing power and memory consumed when denoising the RRI data, or to maintain the overall amount within a desired range.

The processing rules may be applied in a predefined order. For each processing rule, maximum and minimum RRI thresholds may be selected, and RRIs outside of the thresholds may be marked as abnormal. Additionally, each processing rule may be applied at various scales, using sliding analysis windows of a size that is increased to a maximum size. The maximum size may be dynamically determined based on a number of abnormal RRIs marked for removal at each iteration (e.g., at each successive sliding window size). In this way, abnormalities that are detectable at different scales may be removed.

A further advantage of the RRI denoising methods described herein is that abnormal RRIs are not removed upon detection. Rather, the abnormal RRIs are marked for removal when they are detected, and removed at a later stage or final stage. In this way, the marked (abnormal) RRIs can provide context within and/or between stages of the process (e.g., at successively greater sliding window sizes, and in the application of successive rules) for identifying additional abnormalities.

The technical effect of denoising RRI data by marking abnormal RRIs over an iterative process where successive processing rules are applied at increasing scales defined by differently sized sliding analysis windows, is that a quality of the RRI data for performing additional analyses and/or training models to perform inferences on the RRI data may be increased. As a result of the increased quality, the additional analyses may be more accurate, and a performance of the models may be increased with respect to other approaches to denoising RRI data.

The disclosure also provides support for a method for a computational system for denoising physiological cardiac data, the method comprising: extracting R-R interval (RRI) data from the physiological cardiac data, selecting a set of processing rules to apply to the RRI data, selecting a maximum size of a sliding analysis window over which the processing rules are applied to the RRI data, and successively applying the set of processing rules to the RRI data in order, each processing rule applied over different scales that increase as a size of a sliding analysis window is increased, and during the application of each processing rule, marking RRIs that exceed maximum and minimum RRI thresholds as abnormal, removing the RRIs marked as abnormal from the RRI data, and storing the denoised RRI data in a memory of the computational system. In a first example of the method, the physiological cardiac data is acquired from one of an electrocardiograph (ECG) sensor and a photoplethysmographic (PPG) sensor. In a second example of the method, optionally including the first example, the stored denoised RRI data is used to train a state detection model used to detect a psychophysiological state of a human. In a third example of the method, optionally including one or both of the first and second examples, successively applying the set of processing rules to the RRI data in order further comprises: identifying a plurality of RRI sequences of unmarked RRIs in the RRI data, and for each RRI sequence of the plurality of RRI sequences, applying a processing rule to the RRI sequence using sliding analysis windows of increasing sizes, up to the maximum size of the sliding analysis window. In a fourth example of the method, optionally including one or more or each of the first through third examples, the size of the sliding analysis windows is defined by one of: a number of RRIs included in the sliding analysis windows, a duration of the sliding analysis windows in seconds. In a fifth example of the method, optionally including one or more or each of the first through fourth examples, the set of processing rules include: calculating a first mean RRI value of a plurality of RRI values included in a sliding analysis window of the sliding analysis windows, for each RRI in an RRI sequence of the plurality of RRI sequences, marking RRIs of the RRI sequence that are greater than a threshold difference from the first mean RRI value as abnormal. In a sixth example of the method, optionally including one or more or each of the first through fifth examples, the threshold difference is one of an absolute difference and a relative difference expressed as a percentage. In a seventh example of the method, optionally including one or more or each of the first through sixth examples, the set of processing rules includes marking all of the RRIs in the RRI sequence as abnormal if a threshold percentage of RRIs in the RRI sequence are marked as abnormal. In a eighth example of the method, optionally including one or more or each of the first through seventh examples, the set of processing rules includes: for each RRI in an RRI sequence of the plurality of RRI sequences: calculating a second mean RRI value between a preceding consecutive RRI and a subsequent consecutive RRI, and in response to a difference between the RRI and the second mean RRI being greater than either an absolute threshold difference or a relative threshold difference, marking the RRI as abnormal. In a ninth example of the method, optionally including one or more or each of the first through eighth examples, applying the processing rule to the RRI sequence further comprises one of: marking normal RRIs of the RRI sequence that are surrounded by abnormal RRIs as abnormal, marking pairs of normal RRIs of the RRI sequence that are surrounded by abnormal RRIs as abnormal, and marking RRI sequences that are greater than or equal to a threshold length of a window of analysis and surrounded by abnormal RRIs as abnormal.

The disclosure also provides support for a method for training a state detection model to detect a psychophysiological state in a human subject, the method comprising: from each subject of a plurality of human subjects, acquiring physiological cardiac data via a sensor in skin contact with the human subject, extracting an array of R-R interval (RRI) data from the physiological cardiac data, removing RRIs marked as abnormal from the RRI data array via a denoising procedure, the denoising procedure comprising successively applying a set of processing rules to the RRI data array in order, wherein each processing rule is applied over sliding analysis windows that are increased in size, and during the application of each processing rule, RRIs that exceed maximum and minimum RRI thresholds are marked as abnormal, removing the RRIs marked as abnormal from the RRI data array, to create a denoised RRI data array, generating a set of training data from the denoised RRI data array, training the state detection model on the set of training data, and storing the trained state detection model in a memory of a state detection system. In a first example of the method, the sensor is either worn on a body part or integrated into a vehicle. In a second example of the method, optionally including the first example, successively applying the set of processing rules to the RRI data in order further comprises: identifying a plurality of RRI sequences of unmarked RRIs in the RRI data, and for each RRI sequence of the plurality of RRI sequences, applying a processing rule to the RRI sequence using sliding analysis windows of increasing sizes, up to a maximum size of the sliding analysis windows. In a third example of the method, optionally including one or both of the first and second examples, the size of the sliding analysis windows is defined by one of: a number of RRIs included in the sliding analysis windows, a duration of the sliding analysis windows in seconds. In a fourth example of the method, optionally including one or more or each of the first through third examples, the set of processing rules include: calculating a first mean RRI value of a plurality of RRI values included in a sliding analysis window, for each RRI in an RRI sequence of the plurality of RRI sequences, marking RRIs of the RRI sequence that are greater than a threshold difference from the first mean RRI value as abnormal, the threshold difference one of an absolute difference and a relative difference expressed as a percentage. In a fifth example of the method, optionally including one or more or each of the first through fourth examples, the set of processing rules includes marking all of the RRIs in the RRI sequence as abnormal if a threshold percentage of RRIs in the sequence are marked as abnormal. In a sixth example of the method, optionally including one or more or each of the first through fifth examples, the set of processing rules includes: for each RRI in an RRI sequence of the plurality of RRI sequences: calculating a second mean RRI value between a preceding consecutive RRI and a subsequent consecutive RRI, and in response to a difference between the RRI and the second mean RRI being greater than either an absolute threshold difference or a relative threshold difference, marking the RRI as abnormal. In a seventh example of the method, optionally including one or more or each of the first through sixth examples, applying the processing rule to the RRI sequence further comprises one of: marking normal RRIs of the RRI sequence that are surrounded by abnormal RRIs as abnormal, marking pairs of normal RRIs of the RRI sequence that are surrounded by abnormal RRIs as abnormal, and marking RRI sequences that are greater than or equal to a threshold length of a window of analysis and surrounded by abnormal RRIs as abnormal.

The disclosure also provides support for a state detection device of a vehicle, the state detection device configured to detect a psychophysiological state of a driver of a vehicle, the state detection device comprising: a processor and a memory storing instructions that when executed, cause the processor to: acquire physiological cardiac data of a user of the state detection device via one or more sensors of the state detection device, extract a first set of R-R interval (RRI) data from the physiological cardiac data, detect a psychophysiological state of the user based on the first set of RRI data, using a state detection model of the state detection device, in response to detecting the psychophysiological state, adjust one or more controls of the vehicle, wherein the state detection model is trained on training data generated from a second set of RRI data collected from a plurality of drivers, the second set of RRI data denoised via a denoising procedure comprising successively applying a set of processing rules to the second set of RRI data in order, wherein each processing rule is applied over sliding analysis windows that are increased in size, and RRIs that exceed maximum and minimum RRI thresholds are marked as abnormal during the application of each processing rule. In a first example of the system, the set of processing rules include: a first processing rule in which RRIs included in an RRI sequence of the second set of RRI data that are greater than a threshold difference from a mean RRI of the RRI sequence are marked as abnormal, a second processing rule in which all RRIs included in the RRI sequence are marked as abnormal in response to a threshold percentage of the RRIs in the RRI sequence being marked as abnormal, a third processing rule in which an RRI included in the RRI sequence is marked as abnormal in response to the RRI exceeding a threshold difference from a mean RRI value of a preceding consecutive RRI of the RRI and a subsequent consecutive RRI of the RRI, a fourth processing rule in which normal RRIs of the RRI sequence that are surrounded by abnormal RRIs are marked as abnormal, a fifth processing rule in which pairs of normal RRIs of the RRI sequence that are surrounded by abnormal RRIs are marked as abnormal, and a sixth processing rule in which RRI sequences that are greater than or equal to a threshold length of a window of analysis and surrounded by abnormal RRIs are marked as abnormal.

When introducing elements of various embodiments of the present disclosure, the articles "a," "an," and "the" are intended to mean that there are one or more of the elements. The terms "first," "second," and the like, do not denote any order, quantity, or importance, but rather are used to distinguish one element from another. The terms "comprising," "including," and "having" are intended to be inclusive and mean that there may be additional elements other than the listed elements. As the terms "connected to," "coupled to," etc. are used herein, one object (e.g., a material, element, structure, member, etc.) can be connected to or coupled to another object regardless of whether the one object is directly connected or coupled to the other object or whether there are one or more intervening objects between the one object and the other object. In addition, it should be understood that references to "one embodiment" or "an embodiment" of the present disclosure are not intended to be interpreted as excluding the existence of additional embodiments that also incorporate the recited features.

In addition to any previously indicated modification, numerous other variations and alternative arrangements may be devised by those skilled in the art without departing from the spirit and scope of this description, and appended claims are intended to cover such modifications and arrangements. Thus, while the information has been described above with particularity and detail in connection with what is presently deemed to be the most practical and preferred aspects, it will be apparent to those of ordinary skill in the art that numerous modifications, including, but not limited to, form, function, manner of operation and use may be made without departing from the principles and concepts set forth herein. Also, as used herein, the examples and embodiments, in all respects, are meant to be illustrative only and should not be construed to be limiting in any manner.

## Claims

1. A method for a computational system for denoising physiological cardiac data, the method comprising:
extracting R-R interval (RRI) data from the physiological cardiac data;
selecting a set of processing rules to apply to the RRI data;
selecting a maximum size of a sliding analysis window over which the processing rules are applied to the RRI data; and
successively applying the set of processing rules to the RRI data in order, each processing rule applied over different scales that increase as a size of a sliding analysis window is increased, and during the application of each processing rule, marking RRIs that exceed maximum and minimum RRI thresholds as abnormal;
removing the RRIs marked as abnormal from the RRI data; and
storing the denoised RRI data in a memory of the computational system.

2. The method of claim 1, wherein the physiological cardiac data is acquired from one of an electrocardiograph (ECG) sensor and a photoplethysmographic (PPG) sensor.

3. The method of claim 1, wherein the stored denoised RRI data is used to train a state detection model used to detect a psychophysiological state of a human.

4. The method of claim 1, wherein successively applying the set of processing rules to the RRI data in order further comprises:
identifying a plurality of RRI sequences of unmarked RRIs in the RRI data; and
for each RRI sequence of the plurality of RRI sequences, applying a processing rule to the RRI sequence using sliding analysis windows of increasing sizes, up to the maximum size of the sliding analysis window.

5. The method of claim 4, wherein the size of the sliding analysis windows is defined by one of:
a number of RRIs included in the sliding analysis windows; and
a duration of the sliding analysis windows in seconds.

6. The method of claim 4, wherein the set of processing rules include:
calculating a first mean RRI value of a plurality of RRI values included in a sliding analysis window of the sliding analysis windows; and
for each RRI in an RRI sequence of the plurality of RRI sequences, marking RRIs of the RRI sequence that are greater than a threshold difference from the first mean RRI value as abnormal.

7. The method of claim 6, wherein the threshold difference is one of an absolute difference and a relative difference expressed as a percentage.

8. The method of claim 4, wherein the set of processing rules includes marking all of the RRIs in the RRI sequence as abnormal if a threshold percentage of RRIs in the RRI sequence are marked as abnormal.

9. The method of claim 4, wherein the set of processing rules includes:
for each RRI in an RRI sequence of the plurality of RRI sequences:
calculating a second mean RRI value between a preceding consecutive RRI and a subsequent consecutive RRI; and
in response to a difference between the RRI and the second mean RRI being greater than either an absolute threshold difference or a relative threshold difference, marking the RRI as abnormal.

10. The method of claim 4, wherein applying the processing rule to the RRI sequence further comprises one of:
marking normal RRIs of the RRI sequence that are surrounded by abnormal RRIs as abnormal;
marking pairs of normal RRIs of the RRI sequence that are surrounded by abnormal RRIs as abnormal; and
marking RRI sequences that are greater than or equal to a threshold length of a window of analysis and surrounded by abnormal RRIs as abnormal.

11. A state detection device of a vehicle, the state detection device configured to detect a psychophysiological state of a driver of a vehicle, the state detection device comprising:
a processor and a memory storing instructions that when executed, cause the processor to:
acquire physiological cardiac data of a user of the state detection device via one or more sensors of the state detection device;
extract a first set of R-R interval (RRI) data from the physiological cardiac data;
detect a psychophysiological state of the user based on the first set of RRI data, using a state detection model of the state detection device; and
in response to detecting the psychophysiological state, adjust one or more controls of the vehicle;
wherein the state detection model is trained on training data generated from a second set of RRI data collected from a plurality of drivers, the second set of RRI data denoised via a denoising procedure comprising successively applying a set of processing rules to the second set of RRI data in order, wherein each processing rule is applied over sliding analysis windows that are increased in size, and RRIs that exceed maximum and minimum RRI thresholds are marked as abnormal during the application of each processing rule.

12. The state detection device of claim 11, wherein the set of processing rules include:
a first processing rule in which RRIs included in an RRI sequence of the second set of RRI data that are greater than a threshold difference from a mean RRI of the RRI sequence are marked as abnormal;
a second processing rule in which all RRIs included in the RRI sequence are marked as abnormal in response to a threshold percentage of the RRIs in the RRI sequence being marked as abnormal;
a third processing rule in which an RRI included in the RRI sequence is marked as abnormal in response to the RRI exceeding a threshold difference from a mean RRI value of a preceding consecutive RRI of the RRI and a subsequent consecutive RRI of the RRI;
a fourth processing rule in which normal RRIs of the RRI sequence that are surrounded by abnormal RRIs are marked as abnormal;
a fifth processing rule in which pairs of normal RRIs of the RRI sequence that are surrounded by abnormal RRIs are marked as abnormal; and
a sixth processing rule in which RRI sequences that are greater than or equal to a threshold length of a window of analysis and surrounded by abnormal RRIs are marked as abnormal.
